# FASCICULE DE BREVET EUROPEEN

(11) **EP 0 953 015 B1**
(45) Date de publication et mention de la délivrance du brevet: **29.01.2003**
(21) Numéro de dépôt: 97953989.7
(22) Date de dépôt: 31.12.1997
(51) Int. Cl.: C08L 83/12, A61K 7/48, A61K 7/06

(54) **COMPOSITION EPAISSIE AQUEUSE ET UTILISATION**
VERDICKTE, WÄSSRIGE ZUSAMMENSETZUNG UND IHRE ANWENDUNG
THICKENED AQUEOUS COMPOSITION AND USE

(30) Priorité: 14.01.1997 FR 9700294
(43) Date de publication de la demande: 03.11.1999
(73) Titulaire: L'OREAL, 75008 Paris (FR)
(72) Inventeur: DUBIEF, Claude, F-78150 Le Chesnay (FR); DUPUIS, Christine, F-75018 Paris (FR)
(74) Mandataire: Miszputen, Laurent
(86) Numéro de dépôt international: FR9702473
(87) Numéro de publication internationale: WO98031751

(56) Documents cités:
- EP-A- 0 412 706
- EP-A- 0 426 520
- FR-A- 2 709 955
- FR-A- 2 739 282
- FR-A- 2 739 283
- US-A- 5 330 758

## Description

La présente invention a trait à une composition aqueuse comprenant un nouveau système épaississant, ainsi qu'à l'utilisation de ce nouveau système épaississant dans une composition aqueuse susceptible d'être utilisée notamment dans le domaine de la cosmétique.

Il est connu d'utiliser en tant qu'agent épaississant des milieux aqueux, des polymères hydrosolubles ou hydrodispersibles, et notamment des polymères éventuellement réticulés. L'épaississement est alors provoqué par l'enchevêtrement des chaînes de polymères, lesdits polymères amphiphiles ayant de préférence une longueur de chaîne importante et un poids moléculaire élevé.
Il est également connu d'utiliser comme épaississant des milieux aqueux, des polymères hydrophiles comportant des groupements hydrophobes se présentant sous forme de séquences, de greffons et/ou de groupes latéraux répartis de façon aléatoire. Ces polymères amphiphiles permettent d'obtenir un épaississement important du milieu même lorsqu'ils sont utilisés en faible quantité. L'épaississement est généré par la formation d'agrégats entre les groupes hydrophobes du polymère amphiphile, ces agrégats constituant des points de réticulation physique entre les chaînes macromoléculaires.
Toutefois, on a constaté que la présence de polymères hydrophiles à groupements hydrophobes, même en faible quantité, dans des compositions notamment cosmétiques, pouvait modifier de façon indésirable les propriétés cosmétiques desdites compositions, par exemple les propriétés de toucher ou d'étalement.

D'autre part, il est également connu de préparer des compositions capillaires sous forme de gel comprenant des polymères à motifs hydrophobes associés à des tensioactifs; le gel est alors formé grâce à la formation de micelles mixtes.

Toutefois, on a constaté que la texture obtenue était souvent cassante ce qui rendait la composition difficilement préhensile; de plus, la présence d'un excès en tensioactif pouvait conduire à certains inconvénients dans le domaine des compositions non rincées.

La demande de brevet EP 0 412 706 décrit un support particulier pour des compositions cosmétiques et destiné à produire une rhéologie ressemblant à celle d'un gel. Ce véhicule comprend un polymère épaississant et un tensio-actif. Le polymère épaississant est un polymère non-ionique, dont le squelette est soluble dans l'eau et dont les greffons sont hydrophobes. Il peut être par exemple un éther de cellulose rendu hydrophobe. Le tensio-actif peut être une silicone copolyol.
Néanmoins, ce système de véhicule limite la gamme des viscosités des compositions obtenues et ne permet pas d'obtenir des gels d'aspect limpide. Par ailleurs, les propriétés cosmétiques obtenues avec ce support restent insuffisantes, notamment si l'on augmente la concentration en polymères pour augmenter l'épaississement.
Ainsi, il subsiste le besoin d'un système épaississant permettant d'épaissir, voire de gélifier, de manière convenable, dans une très large gamme de viscosités, une composition comprenant une phase aqueuse, sans influer sur les propriétés cosmétiques desdites compositions.

La présente invention a pour but de proposer un tel système épaississant, qui permet de plus d'obtenir un épaississement adéquat en utilisant une très faible quantité de polymère amphiphile épaississant.

Un objet de la présente invention est donc une composition aqueuse, comprenant l'association d'un polymère amphiphile qui comporte au moins une chaîne grasse et des motifs hydrophiles, et d'une silicone polyoxyalkylénée, le polymère amphiphile n'étant pas un éther de cellulose rendu hydrophobe.

Un autre objet de l'invention est l'utilisation de l'association d'une silicone polyoxyalkylénée et d'un polymère amphiphile qui comporte au moins une chaîne grasse et des motifs hydrophiles, en tant qu'agent épaississant, notamment dans une composition comprenant une phase aqueuse, le polymère amphiphile n'étant pas un éther de cellulose rendu hydrophobe.

Enfin un autre objet de l'invention est l'utilisation d'une silicone polyoxyalkylénée pour améliorer le pouvoir épaississant d'un polymère amphiphile qui comporte au moins une chaîne grasse et des motifs hydrophiles dans une composition comprenant une phase aqueuse, le polymère amphiphile n'étant pas un éther de cellulose rendu hydrophobe.

On a donc constaté qu'une telle association permettait d'obtenir, en milieu aqueux, une forte augmentation de la viscosité pouvant aller jusqu'à la gélification totale du milieu.
Il est donc possible de parvenir au même niveau d'épaississement, voire de gélification, en utilisant des quantités plus faibles de polymère amphiphile, tout en conservant de bonnes propriétés cosmétiques. Il est à noter que selon le polymère amphiphile et les quantités auxquelles il est utilisé en présence de silicone, son utilisation en absence de silicone polyoxyalkylénée ne permet pas forcément d'obtenir un épaississement du milieu.

L'interaction silicone polyoxyalkylénée/polymère amphiphile permet donc de régler très facilement le degré de viscosité du milieu par simple mélange, dans des proportions ajustables à volonté.

D'autre part, la composition cosmétique, et notamment capillaire, obtenue, s'étale aisément, présente une meilleure préhension, ainsi qu'une bonne élimination au rinçage.

Sans être tenu par cette explication, on peut considérer que, dans le cadre de l'invention, l'augmentation de la viscosité du milieu résulte d'une réticulation physique entre les chaînes de polymère et la silicone, ladite réticulation étant réversible et faisant intervenir des associations ou interactions de type hydrophobe entre, d'une part, les groupes hydrophobes du polymère amphiphile et d'autre part, les sites hydrophobes de la silicone.

Ces interactions de type hydrophobe conduisent alors au réseau de gélification.

Les polymères susceptibles d'être utilisés dans la présente invention sont des polymères amphiphiles qui comportent au moins une chaîne grasse, donc une partie hydrophobe, et des motifs hydrophiles, donc une partie hydrophile.

La partie hydrophobe peut être en nombre réduit vis-à-vis du reste de la chaîne polymérique, et peut se situer latéralement à la chaîne et être répartie de façon aléatoire (copolymères statistiques) ou répartie sous forme de séquences ou de greffons (copolymères blocs ou copolymères séquences).
On peut utiliser des polymères solubles dans l'eau, ou hydrodispersibles. De préférence, les polymères amphiphiles utiles selon l'invention ne sont pas réticulés.

Les polymères amphiphiles peuvent être de toute nature chimique; on peut ainsi choisir des polymères naturels, éventuellement modifiés; des polymères radiculaires notamment vinyliques ou acryliques; des polycondensats; et leurs mélanges.
Ils peuvent être ioniques ou non ioniques, et sont de préférence anioniques ou non ioniques.

Par l'expression "chaîne - hydrophobe" on doit entendre selon l'invention, un groupement hydrocarboné linéaire ou ramifiée ayant de 8 à 28 atomes de carbone et pouvant comprendre des hétéroatomes tels l'oxygène, l'azote ou le soufre.

On peut en particulier citer, parmi les polymères amphiphiles selon l'invention les polymères dérivés de polymères naturels suivants :
- les celluloses cationiques quaternisées modifiées par des groupements comportant au moins une chaîne grasse tels que des groupes alkyle, arylalkyle, alkylaryle ou leurs mélanges où les groupes alkyle sont de préférence en C₈-C₂₂, notamment les alkylhydroxyéthylcelluloses quaternisées telles que les produits QUATRISOFT LM 200, QUATRISOFT LM-X 529-18-A, QUATRISOFT LM-X 529-18-B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C₁₈) commercialisés par la société CRODA;
- les galactomannanes possédant des substituants hydrophobes, et notamment les gommes de guar substituées hydrophobe ( notamment ceux décrits dans EP281360) et les gommes d'hydroxypropylguars modifiés par des groupements comportant au moins une chaîne grasse tel que le produit ESAFLOR HM 22 (chaîne alkyle en C₂₂) commercialisé par la société LAMBERTI, les produits MIRACARE XC95-3 (chaîne alkyle en C₁₄) et RE205-1 (chaîne alkyle en C₂₀) commercialisés par la société RHONE POULENC ;
- des pullulanes modifiés par des groupes hydrophobes, en particulier des groupes cholestérol ;
- des gélatines modifiées par des groupes hydrophobes, et notamment modifiées par des groupes alkyles en C₆ à C₁₈;
- des muccopolysaccharides tels que ceux constitués de glycosaminoglycane et d'acide hyaluronique.

Parmi les polycondensats utiles selon l'invention, on peut citer les polyuréthannes associatifs qui sont des copolymères séquences non ioniques comportant dans la chaîne, à la fois des séquences hydrophiles de nature le plus souvent polyoxyéthylène et des séquences hydrophobes qui peuvent être des enchaînements aliphatiques seuls et/ou des enchaînements cycloaliphatiques et/ou aromatiques.
Les copolymères séquences résultant peuvent être du type tribloc ou multibloc. Les séquences hydrophobes peuvent-donc être chaque extrémité de la chaîne (copolymères triblocs à séquence centrale polyoxyéthylène) ou réparties à la fois aux extrémités et dans la chaîne (copolymères multiséquencés). Ils peuvent être également en greffons ou en étoile.
On peut citer les polymères décrits dans l'article de ZEYING MA, J. of Appl. Polymer Sci, vol. 49, 1509-27 (1993).
Parmi les polymères commerciaux, on peut citer les produits SER-AD FX1100 et SER-AD FX1035 commercialisés par la société HOLS.

Parmi les polymères amphiphiles radiculaires utiles selon l'invention, on peut citer les polymères acryliques anioniques, en dispersion aqueuse, désignés généralement sous le nom de HASE (hydrophobically modified alkalisoluble or swellable emulsion).
Ce sont des copolymères acryliques existant sous forme de dispersions dans l'eau à pH acide et qui peuvent se solubiliser dans l'eau par neutralisation complète des groupes anioniques, c'est-à-dire au-delà de pH 8.
Ces copolymères sont généralement des terpolymères entre un monomère porteur de groupe acide carboxylique (acide acrylique, méthacrylique), un monomère relativement insoluble à l'eau du type acrylate ou méthacrylate en C₁ à C₄, tel que l'acrylate d'éthyle, et un troisième monomère porteur d'un groupe hydrophobe, qui peut être rattaché latéralement à la chaîne principale. Ce groupe hydrophobe peut être un groupe alkyle linéaire ou ramifié en C₈-C₂₂ et/ou un groupe cycloalkyle en C₈-C₂₂ et/ou un groupe aryle. Le groupe hydrophobe peut être rattaché à la chaîne principale directement par l'intermédiaire d'une liaison éther, ester ou amide, carbamate ou urée. Il peut également être rattaché à la chaîne principale par l'intermédiaire d'une séquence polyoxyéthylénée, elle-même fixée à la chaîne par une liaison éther, ester, amide, carbamate ou urée. Dans ce dernier cas, les groupes latéraux sont généralement de petits greffons à séquence hydrophile et hydrophobe et les propriétés d'épaississement des milieux aqueux sont plus performantes.
De telles dispersions aqueuses de polymère amphiphile sont notamment décrites dans SHAY, (Surface Coatings International, 1993 (11) 446-453), et dans les brevets US4421902, US4423199 et US4663385 de Rohm et Haas, et US4384096 de Dow Corning.
On peut également citer les produits Acusol 823 et les Acrysol 25 ou 22 commercialisés par la société Rohm & Haas.

Parmi les polymères radicalaires selon l'invention, on peut encore citer:
- les copolymères acide acrylique ou acide méthacrytique avec les Nalkylacrylamides, et en particulier, les copolymères acide acrylique/Nalkylacrylamides ayant un groupe alkyle de C₁ à C₂₀ tels que ceux décrits dans l'article MAGNY et al., Double Liaison, 451, p 52-55 (1993). Ils peuvent être obtenus par copolymérisation directe ou par amidification ultérieure de la chaîne d'acide acrylique. Selon le mode opératoire utilisé, les groupes hydrophobes alkyles peuvent être répartis de façon aléatoire (amidification en solution organique homogène) ou sous forme séquencée (amidification en milieu aqueux où l'amine forme initialement des agrégats de type micellaire).
- les copolymères entre un monomère à groupe acide carboxylique, par exemple l'acide méthacrylique, et des méthacrylates d'esters ou d'amides porteurs de groupes hydrophobes cycloaliphatiques ou aromatiques, tels que des groupes isobornyle ou adamantyle.
- les copolymères avec des monomères perfluorés en particulier les copolymères avec le (méthacrylate de perfluorohexyle; des copolymères entre un monomère porteur d'un groupe acide sulfonique (en particulier acide acrylamido-2-méthyl-2 propane suifonique, acide styrène sulfonique) et un alkyle (méth)acrylamide possédant au moins 8 carbones.
- les copolymères acryliques non ioniques, et notamment des copolymères du type acrylamide/N-alkylacrylamide, tels que ceux décrits dans GOODVVIN et al., Polymer in Aqueous Media = Performance Through Association, (J.E. Glassed) Adv. Chem. Ser. 223; Am. Chem. Soc., Washington DC, p 365 (1989).
- les polymères d'acide (méth)acrylique modifiés par des groupes comportant au moins une chaîne grasse ou les copolymères d'acide (méth)acrylique et de monomères comportant au moins une chaîne grasse ; ces monomères sont choisis parmi les monomères hydrophobes à chaîne grasse, les monomères amphiphiles comportant une partie hydrophobe à chaîne grasse et une partie hydrophile ou bien leurs mélanges ; on peut citer à titre d'exemple :
   - les copolymères acide (méth)acrylique/acrylate d'éthyle/acrylate d'alkyle C₈-C₂₂ tels que le produit ACUSOL 823 commercialisé par la société ROHM & HAAS et le produit IMPERON R commercialisé par la société HOECHST;
   - les copolymères acide acrylique/(méth)acrylate de lauryle tels que les produits COATEX SX commercialisés par la société COATEX ;
   - les copolymères acide (méth)acrylique / acrylate d'alkyle en C₁-C₂₂ allyl éther d'alkyle C₁-C₂₂ polyéthoxylé dans lesquels au moins un des contient une chaîne alkyle en C₈-C₂₂ tels que les produits RHEOVIS -CR, -CR3, -CR2 et -CRX commercialisés par la société ALLIED COLLOIDS ;
   - les terpolymères acide méthacrylique/acrylate d'éthyle/acrylate de lauryle polyoxyéthyléné tels que le produit RHEO 2000 commercialisé par COATEX ;
   - les copolymères acide méthacrylique/acrylate d'éthyle/méthacrylate de stéaryle polyoxyéthyléné tels que les produits ACRYSOL 22, ACRYSOL25 et DW-1206A commercialisés par la société ROHM & HAAS ;
   - les copolymères acide méthacrylique/acrylate d'éthyle/acrylate de nonylphénol polyoxyéthyléné tels que le produit RHEO 3000 commercialisé par COATEX;
   - les copolymères acide acrylique/monoitaconate de stéaryle polyoxyéthyléné ou les copolymères acide acrylique/monoitaconate de cétyle polyoxyéthyléné tels que les produits 8069-72A et 8069-72B commercialisés par NATIONAL STARCH;
   - les copolymères acide méthacrylique/acrylate de butyle/monomère hydrophobe comportant une chaîne grasse tels que le produit 8069-146A commercialisé par NATIONAL STARCH;
   - les terpolymères acide acrylique/acrylate d'alkyle en C₈-C₂₀. De préférence en C₁₅/acrylate de polyéthylèneglycol (de préférence de 20 à 30 moles d'oxyde d'éthylène) tels que le produit DAPRAL GE 202 commercialisé par la société AKZO ;
   - les copolymères acide (méth)acryliquelacrylate d'alkyle en C₁-C₂₂ /monomère amphiphile comportant une chaîne hydrocarbonée en C₈-C₂₂ (par exemple alkyle ou alkényle) comprenant des groupements uréthanne tels que le produit ADDITOL VXW 1312 commercialisé par HOECHST;
   - les polymères acryliques modifiés par des groupes hydrophobes à chaîne grasse (chaîne hydrocarbonée en C₈-C₂₂ tels que alkyle ou alkényle) tels que le produit CS-0406 commercialisé par ROHM & HAAS.

Parmi les polymères amphiphiles, on préfère, ceux choisis dans le groupe constitué par :
- les terpolymères acide acrylique/acrylate d'alkyle en C₁-C₁₈/méthacrylate de stéaryle polyoxyéthyléné, par exemple à l'aide de 20 moles d'oxyde d'éthylène tels que le produit commercialisé sous la dénomination de "ACRYSOL ICS-I®" par la Société ROHM & HAAS,
- les terpolymères acide (méth)acrylique/acrylate d'éthyle/ acrylate d'alkyle en C₈-C₂₂, tels que le produit commercialisé sous la dénomination de "IMPERON R®" par la Société HOECHST,
- les terpolymères acide (méth)acrylique/acrylate d'alkyle en C₈-C₂₂/allyl éther d'alkyle C₁-C₂₂ polyéthoxylé dans lesquels au moins un des monomères comprend un alkyle en C₈-C₂₂, tels que les produits commercialisés sous les dénominations de "RHEOVIs-CR®, -CR₂®, -CR₃® et -CRX®" par la SOCIETE ALLIED COLLOIDS,
- les terpolymères acide (méth)acryliquelacrylate d'éthyle/ méthacrylate de stéaryle polyoxyéthyléné, tels que ceux commercialisés sous les dénominations D'"ACRYSOL 25®" et de "DW-1206A®" par la Société ROHM & HAAS.

Selon leur nature, les polymères amphiphiles selon l'invention peuvent être utilisés sous forme de solutions aqueuses ou sous forme de dispersions aqueuses.

On peut indifféremment employer un polymère filmogène ou un polymère non filmogène, voire un mélange de polymère filmogène et non filmogène.

Les compositions selon l'invention comprennent en outre nécessairement au moins une silicone polyoxyalkylénée.

Selon l'invention, on désigne par silicone polyoxyalkylénée toute silicone comportant au moins un groupement oxyalkyléné de type (-CₓH₂ₓO)ₐ dans lequel x peut varier de 2 à 6 et a est supérieur ou égal à 2.
Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnarisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀. et en particulier méthyle, les radicaux fluoroalkyles, les radicaux aryles et en particulier phényle.

Conformément à l'invention, les silicones oxyalkylénées sont choisies parmi les composés de formules générales (I), (II) ou (IV): 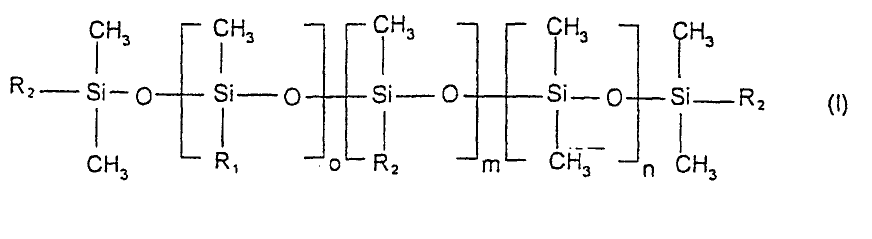 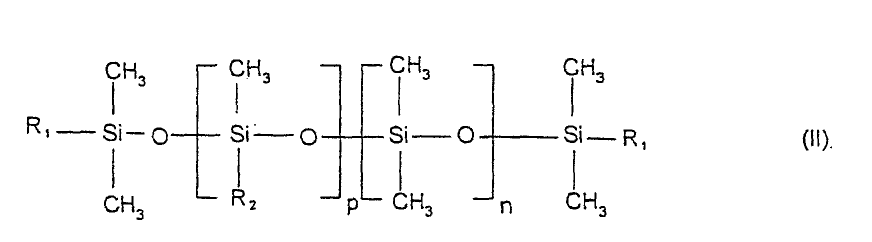 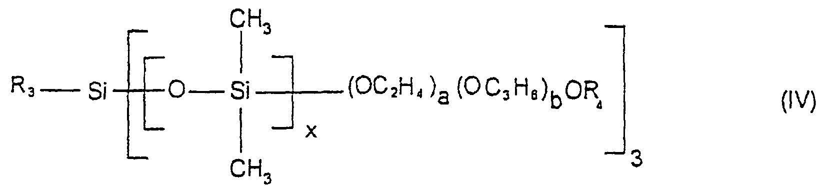 formules (I), (II) et (IV) dans lesquelles:
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C_{1 à} C₁₂, et de préférence le radical méthyle,
- R₅, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'aminé, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO(CH₂)_{d}COOM, -COCHR₇(CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 1 00,
- Y représente un anion minéral ou organique monovalent tel que halogénure (chlorure, bromure), sulfate, carboxylate (acétate, lactate, citrate),
sous réserve que lorsque la silicone est de formule (II) avec R₅ désignant hydrogène alors n est supérieur à 12.

De telles silicones sont par exemple commercialisées par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHONE POULENC sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.
Ces silicones sont notamment décrites dans les brevets US-A-5 070 171, US-A-5149765, US-A-5093452 et US-A-5091493.

Selon un mode particulièrement avantageux de réalisation de la présente invention, la silicone oxyalkylénée répond à la formule (I) dans laquelle m = 0 et satisfait à la formule (III) suivante :

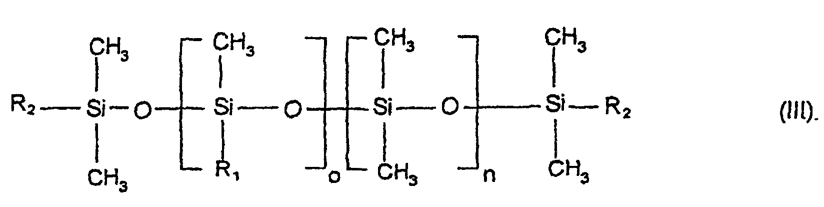

De préférence, on utilise les silicones polyoxyalkyténées répondant aux formules générales (II) ou (III). Plus particulièrement, ces formules répondent à au moins une des, et de préférence toutes les, conditions suivantes :
- c est égal à 2 ou 3.
- R₁ désigne le radical méthyle.
- R₅ représente un radical méthyle, un radical acyle en C₁₂-C₂₂, -CO(CH₂)_{d}COOM.
- a varie de 2 à 25 et plus particulièrement de 2 à 15.
- b est égal à 0.
- n varie de 0 à 100.
- p varie de 1 à 20.

Les silicones selon l'invention peuvent se présenter sous forme de solutions aqueuses ou éventuellement sous forme de dispersions ou d'émulsions aqueuses.

L'association selon l'invention peut en particulier être utilisée pour épaissir, voire gélifier, des milieux aqueux de manière à obtenir, par exemple des gels aqueux.
Elle peut éventuellement être utilisée dans le cadre de l'épaississement d'émulsions, en particulier pour des émulsions exemptes de tensioactifs, ou pour l'épaississement de dispersions aqueuses.
On peut ainsi envisager des applications notamment dans les domaines de la cosmétique, de la dermatologie ou de l'hygiène, pour l'épaississement notamment de gels de soin ou de nettoyage de la peau ou des cheveux, de gels coiffants, de gels solaires, de gels de maquillage et de gels buccodentaires ou pour l'épaississement des émulsions et notamment des émulsions huile-dans-eau, par exemple dans les crèmes de soin, de nettoyage, de maquillage de la peau ou des cheveux, les crèmes solaires, voire capillaires.

De préférence, la composition selon l'invention comprend moins de 5% en poids de tensioactif.

Les quantités de silicone oxyalkylénée et de polymère amphiphile à ajouter à un milieu aqueux pourront également être déterminées par l'homme du métier sur base de ses connaissances générales. On peut notamment envisager une composition comprenant 0,1 à 15% en poids de polymère amphiphile, de préférence de 0,2 à 1 0% en poids par rapport au poids total de la composition.
La quantité de silicone peut être de 0,005% à 15% en poids par rapport au poids total de la composition. De préférence, on utilise les silicones en une quantité telle que l'on obtient un rapport pondéral silicone/polymère amphiphile compris entre 0, 1 et 1 0.
On peut ainsi obtenir une composition épaissie, ayant une viscosité comprise entre 200 et 30 000 cp (mPa.s), et comprenant une quantité très faible d'agents épaississants, pouvant être de l'ordre de 0,8 à 3% en poids par exemple.

Selon l'application envisagée, la composition peut comprendre les constituants habituels à ce type de composition.
On peut citer tout additif usuellement utilisé dans le domaine considéré, tel que les pigments, les charges et/ou les nacres, des antioxydants, des parfums, desconservateurs, des actifs cosmétiques ou pharmaceutiques, des hydratants, des vitamines, des acides gras essentiels, des filtres solaires, des tensioactifs, les autobronzants.
Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

L'invention est illustrée plus en détail dans les exemples suivants.

### Exemple 1

On mesure la viscosité à 25°C d'une composition aqueuse comprenant 1% en poids de matière active de polymère et 0,1% ou 0,5% en poids de matière active de silicone (pH 7 avec de l'amino-2 méthyl-2 propanol-1).

On obtient les résultats suivants :

| Polymère | Silicone | Viscosité |
|---|---|---|
| 1% Acrysol ICS | --- | 1300 cps |
| 1 % Acrysol ICS | 0,1% Fancorsil SLA | 1650 cps |
| 1% Acrysol ICS | 0, 1 % Silwax WDIS | 1700 cps |
| 1% Acrysol ICS | 0,1% Fancorsil LIM-1 | 2400 cps |
| 1% Acrysol ICS | 0,5% Fancorsil LIM-1 | 4300 cps |
| 1% Acrysol ICS | 0, 1 % Silwet L 77 | 2700 cps |
| 1% Acrysol ICS | 0, 1 % Silphos A 1 00 | 1750 cps |
| 1% Acrysol ICS | 0,5% Silphos A 100 | 2400 cps |
| 1% Acrysol ICS | 0,1 % Silicone sulfate | 2080 cps |

Les composés employés sont les suivants
- Acrysol ICS commercialisé par Rohm & Haas :Terpolymère acide acrylique /acrylate d'alkyle en C₁-C₁₈. / méthacrylate de stéaryle polyoxyéthyléné à 20 moles d'oxyde d'éthylène.
- Fancorsil SLA commercialisé par Fanning Corporation : Polydiméthylsiloxane polyoxyéthyléné à groupements adipate
- Silwax WD-IS commercialisé par Siltech : Polydiméthylsiloxane polyoxyéthyléné à groupements stéarate
- Fancorsil LIM-1 commercialisé par Fanning Corporation Polydiméthylsiloxane polyoxyéthyléné à groupements eicosanoate
- Silwet L 77 commercialisé par OSI : Heptaméthyltrisiloxane polyoxyéthyléné à 8 moles d'oxyde d'éthylène
- Silphos A 1 00 commercialisé par Siltech : Polydiméthylsiloxane polyoxyéthyléné à groupements phosphate
- Silicone sulfate commercialisé par Siltech : Polydiméthylsiloxane polyoxyéthyléné à groupements sulfate

On constate donc que l'ajout d'une silicone, à un polymère amphiphile permet d'améliorer le pouvoir épaississant dudit polymère amphiphile alors que la silicone utilisée seule n'apporte aucune viscosité.
De plus, la composition obtenue présente une viscosité adéquate, tout en comprenant une faible quantité d'épaississant.

Ces compositions ont une texture fondante, non cassante.

### Exemple 2

On a préparé un gel de coiffage de composition suivante :
- Terpolymère acide méthacrylique / acrylate d'éthyle / méthacrylate de stéaryle polyoxyéthyléné (ACRYSOL 22 de Rohm & Haas) 2 g
- SILWAX WD IS 0,3 g
- Amino-2 méthyl-2 propanol-1 qs pH 7,5
- Eau déminéralisée qsp 100 g

### Exemple 3

On a préparé un gel de coiffage de composition suivante
- ACRYSOL22 1 g
- SILPHOS A 100 0,5 g
- Amino-2 méthyl-2 propanol-1 qs pH 7,5
- Eau déminéralisée qsp 100 g

### Exemple 4

On a préparé un gel de coiffage de composition suivante :
- Copolymère acide méthacrylique / acrylate d'alkyle en C8-C22 / allyl éther d'alkyle en C1-C22 (RHEOVIS CR de Allîed Colloid) 4 g
- SI LWET L77 0,4 g
- Amino-2 méthyl-2 propanol-1 qs pH 7
- Ethanol 6,8 g
- Eau déminéralisée qsp 100 g

### Exemple 5

On compare ci-après des compositions conformes à l'invention comprenant comme polymère amphiphile, l'Acrysol 22 avec des compositions conformes à l'art antérieur comprenant du Natrosol Plus. On utilise une silicone copolyol en tant que silicone polyoxyalkylénée. On étudie la viscosité ainsi que la réponse cosmétique obtenues pour l'une et l'autre des formulations.

| | A | B | C | D | E | F |
|---|---|---|---|---|---|---|
| Acrysol 22 | 1 | | 1 | | 1 | |
| AMP | 0.35 | | 0.35 | | 0.35 | |
| Natrosol Plus | | 1 | | 1 | | 1 |
| Silwet L77 | | | 0.1 | 0.1 | | |
| Fancorsil LIM | | | | | 0.1 | 0.1 |
| Eau qsp (g) | 100 | 100 | 100 | 100 | 100 | 100 |

### Energie de viscosité

| | A | B | C | D | E | F |
|---|---|---|---|---|---|---|
| Viscosité(cps) | 1060 | 160 | 3400 | 224 | 2030 | 216 |

Les compositions C et E sont nettement supérieures à A. Les compositions D et F sont équivalentes à B.

### Réponse cosmétique: douceur sur mèches SA 20

| | A | B | C | D | E | F |
|---|---|---|---|---|---|---|
| Douceur | 3.00 | 3.25 | 4.00 | 3.50 | 4.25 | 3.00 |

Les compositions C et E sont nettement supérieures à A. Les compositions D et F sont équivalentes à B.

L'utilisation de polymères tels que l'Acrysol 22 conformément à l'invention permet d'obtenir des gels de viscosité élevée et qui sont limpides. Par association avec les silicones définies dans l'invention, on obtient une synergie de viscosité qui ne se produit pas avec le Natrosol Plus. Ces silicones conduisent à une amélioration cosmétique qui ne se produit pas avec le Natrosol Plus. Les compositions conformes à l'invention possèdent donc une viscosité, une limpidité et un effet cosmétique améliorés par rapport aux compositions de l'art antérieur.

## Revendications

1. Composition comprenant, dans une phase aqueuse, l'association d'un polymère amphiphile qui comporte au moins une chaîne grasse, c'est-à-dire un groupement hydrocarboné linéaire ou ramifié ayant de 8 à 28 atomes de carbone pouvant comprendre des hétéroatomes tels que l'oxygène, l'azote ou le soufre, et des motifs hydrophiles, sous réserve que ce polymère amphiphile ne soit pas un éther de cellulose rendu hydrophobe, et d'une silicone polyoxyalkylénée choisie parmi les composés de formules générales (I), (II) ou (IV) : 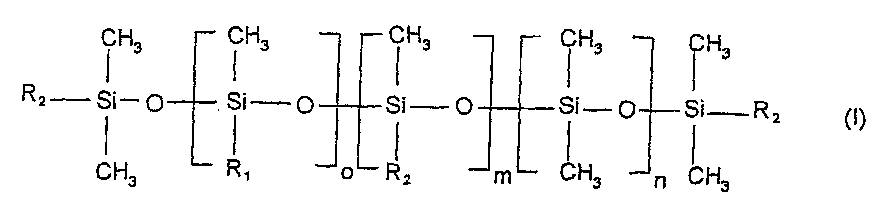 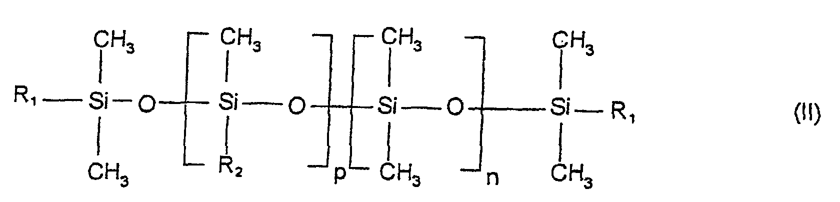 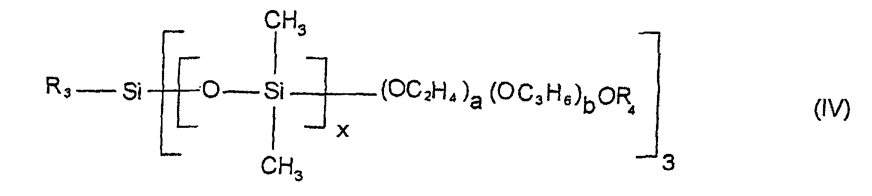 formules (I), (II) ou (IV) dans lesquelles
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅
- R₃, R₄, identiques ou différents, désignent un radical alkyle, linéaire ou ramifié, en C₁-C₁₂, et de préférence le radical méthyle,
- Rs, identique ou différent, est choisi parmi un atome d'hydrogène, un radical alkyle, linéaire ou ramifié, de 1 à 12 atomes de carbone, un radical alcoxy, linéaire ou ramifié, de 1 à 6 atomes de carbone, un radical acyle, linéaire ou ramifié, de 2 à 30 atomes de carbone, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO(CH₂)_{d}COOM, -COCHR₇(CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 1 0,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 1 00,
- Y représente un anion minéral ou organique monovalent,
sous réserve que lorsque la silicone est de formule (II) avec R₅ désignant hydrogène alors n est supérieur à 12.

2. Composition selon la revendication 1, **caractérisée par le fait que** la silicone oxyalkylénée répond à la formule (I) dans laquelle m = 0 et satisfait à la formule (III) suivante :

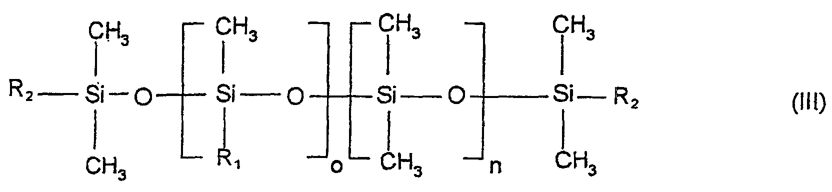

3. Composition selon la revendication 1 ou 2, dans laquelle le polymère amphiphile est choisi parmi les polymères naturels, éventuellement modifiés, les polymères amphiphiles radiculaires, les polycondensats et leurs mélanges.

4. Composition selon l'une des revendications précédentes, dans laquelle le polymère amphiphile est choisi parmi les polymères anioniques ou non ioniques.

5. Composition selon l'une des revendications précédentes, **caractérisée en ce qu'**elle comprend moins de 5% en poids de tensioactif par rapport au poids total de la composition.

6. Composition selon l'une des revendications précédentes, **caractérisée en ce qu'**elle comprend de 0,1 à 15% en poids de polymère amphiphile, de préférence 0,2 à 1 0% par rapport au poids total de la composition.

7. Composition selon l'une des revendications précédentes, **caractérisée en ce qu'**elle comprend de 0,005% à 15% en poids de silicones polyoxyalkylénées, par rapport au poids total de la composition.

8. Composition selon l'une des revendications précédentes, **caractérisée en ce que** le rapport pondéral silicone/polymère amphiphile est compris entre 0, 1 et 10.

9. Composition selon l'une des revendications précédentes, **caractérisée en ce qu'**elle a une viscosité comprise entre 200 et 30 000 cp (mPa.s).

10. Composition selon l'une des revendications précédentes, **caractérisée en ce qu'**elle se présente sous la forme d'un gel aqueux ou d'une émulsion.

11. Utilisation de l'association d'une silicone polyoxyalkylénée choisie parmi les composés de formulas générales (I), (II) ou (IV) et d'un polymère amphiphile, sous réserve que ce polymère amphiphile ne soit pas un éther de cellulose rendu hydrophobe, en tant qu'agent épaississant d'une composition comprenant une phase aqueuse.

12. Utilisation d'une silicone polyoxyalkylénée choisie parmi les composés de formules générales (I), (II) ou (IV) pour améliorer le pouvoir épaississant d'un polymère amphiphile qui comporte au moins une chaîne grasse et des motifs hydrophiles dans une composition comprenant une phase aqueuse, sous réserve que ce polymère amphiphile ne soit pas un éther de cellulose rendu hydrophobe.

## Claims

1. Composition comprising, in an aqueous phase, the combination of an amphiphilic polymer which contains at least one fatty chain, that is to say a linear or branched hydrocarbon group having from 8 to 28 carbon atoms which can comprise heteroatoms, such as oxygen, nitrogen or sulphur, and hydrophilic units, provided that this amphiphilic polymer is not a cellulose ether which has been made hydrophobic, and of a polyoxyalkylenated silicone chosen from the compounds of general formulae (I), (II) or (IV): 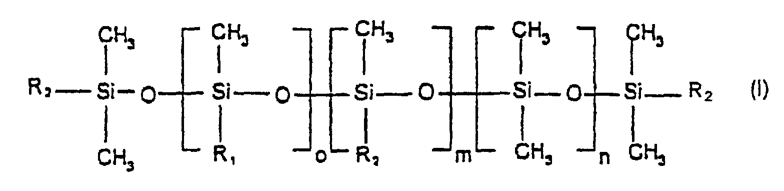 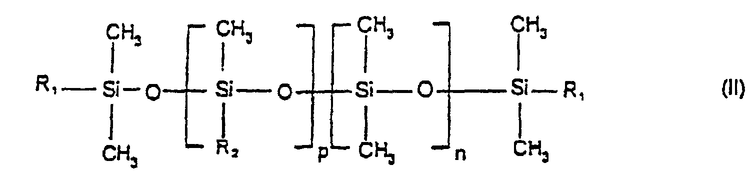 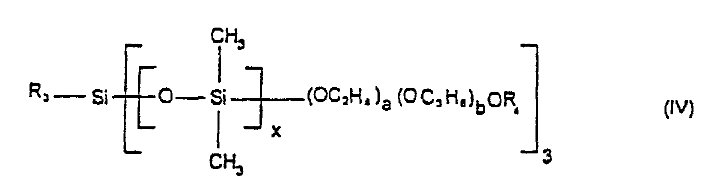 in which formulae (I), (II) or (IV):
- R₁, which is identical or different, represents a linear or branched C₁-C₃₀ alkyl or phenyl radical,
- R₂, which is identical or different, represents a radical -C_{c}H_{2c}-O-(C₂H₄O)ₐ(C₃H₆O)_{b}-R₅ or a radical -C_{c}H_{2c}-O-(C₄H₈O)ₐ-R₅,
- R₃, R₄, which are identical or different, designate a linear or branched C₁-C₁₂ alkyl radical and preferably the methyl radical,
- R₅, which is identical or different, is chosen from a hydrogen atom, a linear or branched alkyl radical having 1 to 12 carbon atoms, a linear or branched alkoxy radical having 1 to 6 carbon atoms, a linear or branched acyl radical having 2 to 30 carbon atoms, a radical -SO₃M, a C₁-C₆ aminoalkoxy radical optionally substituted on the amine, a C₂-C₆ aminoacyl radical optionally substituted on the amine, a radical -NHCH₂CH₂COOM, a radical -N(CH₂CH₂COOM)₂, an aminoalkyl radical optionally substituted on the amine and on the alkyl chain, a C₂-C₃₀ carboxyacyl radical, a phosphono group optionally substituted with one or two substituted aminoalkyl radicals, a radical -CO(CH₂)_{d}COOM, a radical -COCHR₇(CH₂)_{d}COOM, a radical -NHCO(CH₂)_{d}OH, a radical -NH₃Y, a phosphate group,
- M, which is identical or different, designates a hydrogen atom, Na, K, Li, NH₄ or an organic amine,
- R₇ designates a hydrogen atom or a radical SO₃M,
- d varies from 1 to 10,
- m varies from 0 to 20,
- n varies from 0 to 500,
- o varies from 0 to 20,
- p varies from 1 to 50,
- a varies from 0 to 50,
- b varies from 0 to 50,
- a + b is greater than or equal to 2,
- c varies from 0 to 4,
- x varies from 1 to 100,
- Y represents a monovalent inorganic or organic anion, provided that when the silicone is of the formula (II) with R₅ designating hydrogen then n is greater than 12.

2. Composition according to Claim 1, **characterized in that** the oxyalkylenated silicone corresponds to formula (I), in which m = o and satisfies the following formula (III):

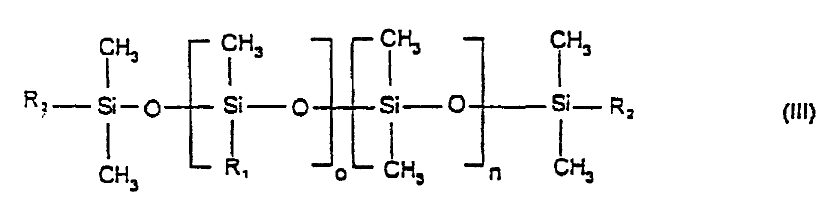

3. Composition according to Claim 1 or 2, in which the amphiphilic polymer is chosen from optionally modified natural polymers, free-radical amphiphilic polymers, polycondensates and the mixtures thereof.

4. Composition according to one of the preceding claims, in which the amphiphilic polymer is chosen from anionic or nonionic polymers.

5. Composition according to one of the preceding claims, **characterized in that** it comprises less than 5% by weight of surfactant relative to the total weight of the composition.

6. Composition according to one of the preceding claims, **characterized in that** it comprises from 0.1 to 15% by weight of amphiphilic polymer, preferably 0.2 to 10% relative to the total weight of the composition.

7. Composition according to one of the preceding claims, **characterized in that** it comprises from 0.005% to 15% by weight of polyoxyalkylenated silicones relative to the total weight of the composition.

8. Composition according to one of the preceding claims, **characterized in that** the silicone/amphiphilic polymer weight ratio is between 0.1 and 10.

9. Composition according to one of the preceding claims, **characterized in that** it has a viscosity of between 200 and 30,000 cp (mPa.s)

10. Composition according to one of the preceding claims, **characterized in that** it is provided in the form of an aqueous gel or of an emulsion.

11. Use of the combination of a polyoxyalkylenated silicone chosen from the compounds of general formulae (I), (II) or (IV) and of an amphiphilic polymer, provided that this amphiphilic polymer is not a cellulose ether which has been made hydrophobic, as thickening agent for a composition comprising an aqueous phase.

12. Use of a polyoxyalkylenated silicone chosen from the compounds of general formulae (I), (II) or (IV), for enhancing the thickening power of an amphiphilic polymer which contains at least one fatty chain and hydrophilic units in a composition comprising an aqueous phase, provided that this amphiphilic polymer is not a cellulose ether which has been made hydrophobic.

## Patentansprüche

1. Zusammensetzung, die in einer wäßrigen Phase ein amphiphiles Polymer, das mindestens eine Fettkette, d.h. eine geradkettige oder verzweigte Kohlenwasserstoffgruppe mit 8 bis 28 Kohlenstoffatomen, die Heteroatome, wie Sauerstoff, Stickstoff oder Schwefel enthalten kann, und hydrophile Einheiten aufweist, mit der Maßgabe, daß das amphiphile Polymer kein hydrophobisierter Celluloseether ist, in Kombination mit einem polyalkoxylierten Silicon enthält, das unter den Verbindungen der allgemeinen Formeln (I), (II) oder (IV) ausgewählt ist: 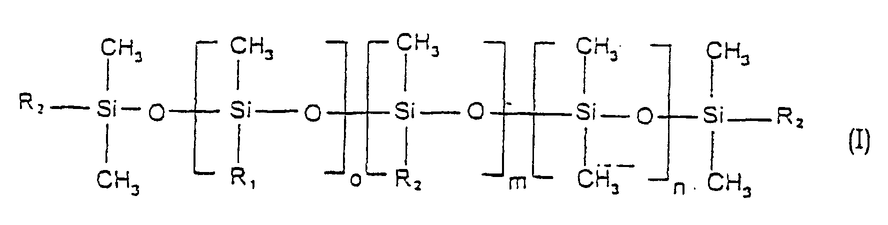 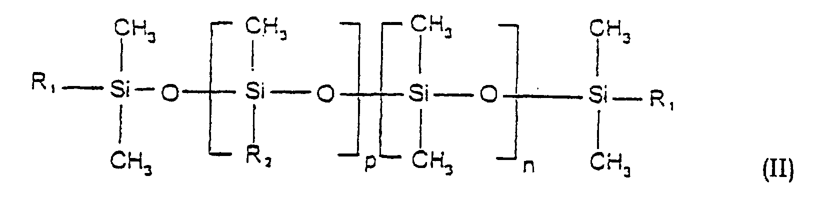 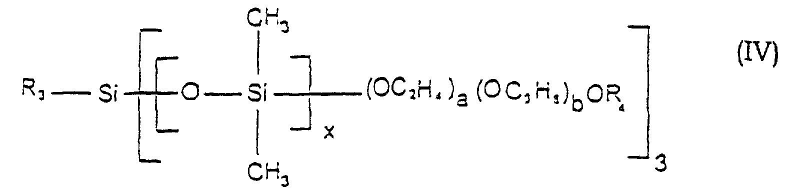 wobei in den Formeln (I), (II) oder (IV):
- die Gruppen R₁, die identisch oder voneinander verschieden sind, eine geradkettige oder verzweigte C₁₋₃₀-Alkylgruppe oder eine Phenylgruppe bedeuten,
- die Gruppen R₂, die identisch oder voneinander verschieden sind, eine Gruppe -C_{c}H_{2c}-O-(C₂H₄O)ₐ(C₃H₆O)_{b}-R₅ oder eine Gruppe -C_{c}H_{2c}-O-(C₄H₈O)ₐ-R₅ bedeuten,
- die Gruppen R₃ und R₄, die identisch oder voneinander verschieden sind, eine geradkettige oder verzweigte C₁₋₁₂-Alkylgruppe und vorzugsweise Methyl bedeuten,
- die Gruppen R₅, die identisch oder voneinander verschieden sind, unter einem Wasserstoffatom, einer geradkettigen oder verzweigten Alkylgruppe mit 1 bis 12 Kohlenstoffatomen, einer geradkettigen oder verzweigten Alkoxygruppe mit 1 bis 6 Kohlenstoffatomen, einer geradkettigen oder verzweigten Acylgruppe mit 2 bis 30 Kohlenstoffatomen, -SO₃M, einer gegebenenfalls an der Aminogruppe substituierten C₁₋₆-Aminoalkoxygruppe, einer gegebenenfalls an der Aminogruppe substituierten C₂₋₆-Aminoacylgruppe, -NHCH₂CH₂COOM, -N(CH₂CH₂COOM)₂, einer gegebenenfalls an der Aminogruppe und an der Alkylgruppe substituierten Aminoalkylgruppe, einer C₂₋₃₀-Carboxyacylgruppe, einer gegebenenfalls mit einer oder zwei substituierten Aminoalkylgruppen substituierten Phosphonogruppe, -CO(CH₂)_{d}COOM, -COCHR₇(CH₂)_{d}COOM, -NHCO(CH₂)_{d}OH, -NH₃Y oder einer Phosphatgruppe ausgewählt sind,
- die Gruppen M, die identisch oder voneinander verschieden sind, Wasserstoff, Na, K, Li, NH₄ oder ein organisches Amin bedeuten,
- R₇ Wasserstoff oder SO₃M ist,
- d im Bereich von 1 bis 10 liegt,
- m im Bereich von 0 bis 20 liegt,
- n im Bereich von 0 bis 500 liegt,
- o im Bereich von 0 bis 20 liegt,
- p im Bereich von 1 bis 50 liegt,
- a im Bereich von 0 bis 50 liegt,
- b im Bereich von 0 bis 50 liegt,
- a + b mindestens 2 bedeutet,
- c im Bereich von 0 bis 4 liegt,
- x im Bereich von 1 bis 100 liegt, und
- Y ein einwertiges anorganisches oder organisches Anion bedeutet,
mit der Maßgabe, daß n über 12 liegt, wenn das Silicon die Formel (II) mit R₅ = Wasserstoff bedeutet.

2. Zusammensetzung nach Anspruch 1, **dadurch gekennzeichnet, daß** das alkoxylierte Silicon der Formel (I) mit m = 0 entspricht und die folgende Formel (III) aufweist:

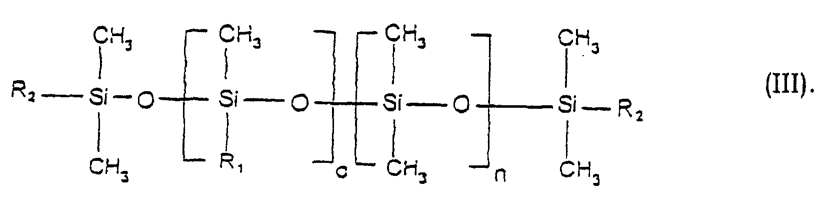

3. Zusammensetzung nach Anspruch 1 oder 2, wobei das amphiphile Polymer unter den natürlichen, gegebenenfalls modifizierten Polymeren, den radikalisch polymerisierbaren amphiphilen Polymeren, den Polykondensaten und deren Gemischen ausgewählt ist.

4. Zusammensetzung nach einem der vorhergehenden Ansprüche, wobei das amphiphile Polymer unter den anionischen oder nichtionischen Polymeren ausgewählt ist.

5. Zusammensetzung nach einem der vorhergehenden Ansprüche, **dadurch gekennzeichnet, daß** sie weniger als 5 Gew.-% grenzflächenaktiven Stoff, bezogen auf das Gesamtgewicht der Zusammensetzung, enthält.

6. Zusammensetzung nach einem der vorhergehenden Ansprüche, **dadurch gekennzeichnet, daß** sie 0,1 bis 15 Gew.-% amphiphiles Polymer und vorzugsweise 0,2 bis 10 %, bezogen auf das Gesamtgewicht der Zusammensetzung, enthält.

7. Zusammensetzung nach einem der vorhergehenden Ansprüche, **dadurch gekennzeichnet, daß** sie 0,005 bis 15 Gew.-% polyalkoxylierte Silicone, bezogen auf das Gesamtgewicht der Zusammensetzung, enthält.

8. Zusammensetzung nach einem der vorhergehenden Ansprüche, **dadurch gekennzeichnet, daß** das Gewichtsverhältnis Silicon/amphiphiles Polymer im Bereich von 0,1 bis 10 liegt.

9. Zusammensetzung nach einem der vorhergehenden Ansprüche, **dadurch gekennzeichnet, daß** sie eine Viskosität im Bereich von 200 bis 30 000 cP (mPa·s) aufweist.

10. Zusammensetzung nach einem der vorhergehenden Ansprüche, **dadurch gekennzeichnet, daß** sie in Form eines wäßrigen Gels oder einer Emulsion vorliegt.

11. Verwendung der Kombination eines polyalkoxylierten Silicons, das unter den Verbindungen der allgemeinen Formeln (I), (II) oder (IV) ausgewählt ist, und eines amphiphilen Polymers, mit der Maßgabe, daß das amphiphile Polymer kein hydrophobisierter Celluloseether ist, als Verdickungsmittel in einer Zusammensetzung, die eine wäßrige Phase enthält.

12. Verwendung eines polyalkoxylierten Silicons, das unter den Verbindungen der allgemeinen Formeln (I), (II) oder (IV) ausgewählt ist, zur Verbesserung des Verdickungsvermögens eines amphiphilen Polymers, das mindestens eine Fettkette und hydrophile Einheiten aufweist, in einer Zusammensetzung, die eine wäßrige Phase enthält, mit der Maßgabe, daß das amphiphile Polymer kein hydrophobisierter Celluloseether ist.
